(19) Europäisches Patentamt
European Patent Office
Office européen des brevets

(11) Veröffentlichungsnummer: **0 328 955 B1**

(12) **EUROPÄISCHE PATENTSCHRIFT**

(45) Veröffentlichungstag der Patentschrift: **03.05.95**

(51) Int. Cl.⁶: **C07D 233/90**, C07D 235/24

(21) Anmeldenummer: **89101847.5**

(22) Anmeldetag: **03.02.89**

# (54) Verfahren zur Herstellung von Imidazol-2-carbonsäuren.

(30) Priorität: **13.02.88 DE 3804545**

(43) Veröffentlichungstag der Anmeldung:
**23.08.89 Patentblatt 89/34**

(45) Bekanntmachung des Hinweises auf die Patenterteilung:
**03.05.95 Patentblatt 95/18**

(84) Benannte Vertragsstaaten:
**BE CH DE FR GB IT LI NL**

(56) Entgegenhaltungen:
**EP-A- 0 172 407**
**DE-A- 2 634 053**
**DE-B- 1 033 667**

(73) Patentinhaber: **BASF Aktiengesellschaft**
**Carl-Bosch-Strasse 38**
**D-67063 Ludwigshafen (DE)**

(72) Erfinder: **Kempe, Uwe, Dr.**
**Danziger Strasse 9**
**D-6701 Dannstadt-Schauernheim (DE)**
Erfinder: **Dockner, Toni, Dr.**
**Grossgasse 6**
**D-6701 Meckenheim (DE)**
Erfinder: **Koehler, Hermann, Dr.**
**Roentgenstrasse 7**
**D-6711 Beindersheim (DE)**

EP 0 328 955 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von 4,5-disubstituierten Imidazol-2-carbonsäuren, indem man 4,5-disubstituierte Imidazole mit Kohlendioxid unter erhöhtem Druck bei Temperaturen von 150 bis 300 °C in Gegenwart einer Base umsetzt.

Ferner betrifft die vorliegende Erfindung neue 4,5-disubstituierte Imidazol-2-carbonsäuren.

Aus der Literatur sind folgende Wege zur Herstellung von Benzimidazol-2-carbonsäuren bekannt:

- Kondensation von o-Phenylendiamin mit Glykolsäure zu 2-Hydroxy-methylbenzimidazol und nachfolgende Oxidation mit $KMnO_4$ (A. Bistrzycki, G. Przeworski, Chem. Ber. 45, 3483 (1912)) und
- Kondensation von 2-Methylbenzimidazol mit Benzaldehyd zur Styryl-verbindung und nachfolgende Oxidation mit $KMnO_4$ (S. Roseman, J. Amer. Chem. Soc.: 75, 3854 (1953)).

Aus der DE-A-1 033 667 ist die Reaktion von Imidazol mit $CO_2$ unter Druck bekannt, die zu Imidazol-4-(5)-carbonsäure bzw. Imidazol-4,5-dicarbonsäureführt. Aus der EP-A-172 407 ist die analoge Reaktion mit in 4- und 5-Stellung unsubstituierten und verschiedenen in 4(5)-Stellung monosubstituierten Imidazolverbindungen bekannt. Dabei tritt die Carboxylgruppe in allen Fällen nur in Position 4 (bzw. 5) des Imidazolringes ein.

Neben den aus den vorstehenden Dokumenten bekannten Benzimidazolen sind aus der DE-A-26 10 527, der DE-A-26 34 053, Tetrahedron Letters 27, 1635-1638 (1986) und J. Heterocycl. Chem. 17, 409-411 (1980) 4,5-Dichlor- bzw. 4,5-Dibromimidazol-2-carbonsäure bekannt.

Der Erfindung lag daher die Aufgabe zugrunde, einen neuen bzw. besseren Zugang zu den 4,5-disubstituierten Imidazol-2-carbonsäuren zu finden.

Demgemäß wurde ein neues Verfahren zur Herstellung von 4,5-disubstituierten Imidazol-2-carbonsäuren der allgemeinen Formel I

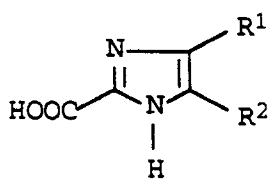 (I),

in der die Substituenten

$R^1$ und $R^2$ unabhängig voneinander $C_1$-$C_{20}$-Alkyl, $C_2$-$C_{20}$-Alkoxyalkyl, $C_3$-$C_{12}$-Cycloalkyl, Halogen, gegebenenfalls durch $C_1$-$C_4$-Alkyl, $C_1$-$C_4$-Alkyl, $C_1$-$C_4$-Alkoxy, Halogen, C1-C4-Halogenalkyl, $C_1$-$C_4$-Halogenalkoxy oder Phenoxy substituiertes Phenyl oder $C_7$-$C_{20}$-Phenylalkyl oder gemeinsam eine gegebenenfalls durch $C_1$-$C_4$-Alkyl, $C_1$-$C_4$-Alkoxy und/oder Halogen ein- bis zweifach substituierte $(CH_2)_n$- oder $(CH=CH)_m$-Gruppe, in der n für 1 bis 6 und m für 1 bis 3 steht,

bedeuten, gefunden, welches dadurch gekennzeichnet ist, daß man 4,5-disubstituierte Imidazole der allgemeinen Formel II

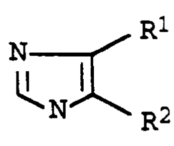 (II),

in der $R^1$ und $R^2$ die obengenannten Bedeutungen haben, mit Kohlendioxid bei Temperaturen von 150 bis 300 °C und Drücken von 10 bis 300 bar in Gegenwart von anorganischen Carbonaten, Hydrogencarbonaten und/oder Hydroxiden als Base umsetzt.

Die 4,5-disubstituierten Imidazol-2-carbonsäuren sind nach folgender Methode erhältlich:

Die Umsetzung erfolgt zwischen einem 4,5-disubstituierten Imidazol und Kohlendioxid unter erhöhtem Druck bei Temperaturen von 150 bis 300 °C in Gegenwart von anorganischen Carbonaten, Hydrogencarbonaten und/oder Hydroxiden als Base nach folgender Reaktionsgleichung:

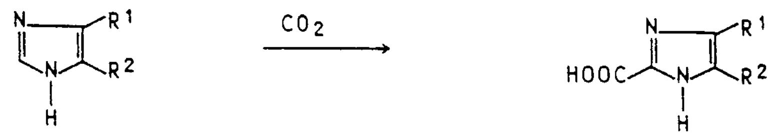

Die Umsetzungen werden vorzugsweise bei Temperaturen von 150 bis 300 °C. besonders bevorzugt von 180 bis 280 °C und bei Drücken zwischen 10 und 300 bar, vorzugsweise bei 10 bis 180 bar, besonders bevorzugt bei 40 bis 180 bar durchgeführt.

Das Verfahren kann kontinuierlich oder diskontinuierlich durchgeführt werden.

Im allgemeinen arbeitet man unter Druck und in Abwesenheit eines Lösungsmittels.

Als anorganische Carbonate, Hydrogencarbonate und/oder Hydroxide eignen sich bevorzugt Alkalimetallhydrogencarbonate, Alkali- und Erdalkalimetallcarbonate und/oder Alkali- und Erdalkalimetall-hydroxide, besonders bevorzugt Kaliumcarbonat.

4,5-disubstituierte Imidazole sind überwiegend bekannt und können nach den in K. Hofmann, Imidazole and its Derivatives, Interscience, New York 1953, S. 33 ff beschriebenen Methoden hergestellt werden.

Das Molverhältnis Kohlendioxid : 4,5-disubstituiertem Imidazol beträgt von 1:1 bis 100:1, vorzugsweise 1:1 bis 10:1.

Das Kohlendioxid kann z.B. gasförmig, flüssig oder fest eingesetzt werden. Vorteilhaft arbeitet man mit flüssigem Kohlendioxid.

Die Reaktion kann jedoch auch in Gegenwart eines Lösungs- oder Verdünnungsmittels durchgeführt werden.

Als Lösungsmittel oder Verdünnungsmittel eignen sich beispielsweise aliphatische Kohlenwasserstoffe wie n-Pentan, n-Hexan, das Hexan-Isomerengemisch und Petrolether; halogenierte Kohlenwasserstoffe wie Chlorbenzol, Methylenchlorid, Ethylenchlorid, Chloroform, Tetrachlorethylen; aromatische Kohlenwasserstoffe wie Benzol, Toluol, die Xylole und deren Isomerengemische, Benzin; Ether wie Diethyl-, Di-n-butylether, Methyl-tert.-butylether, Tetrahydrofuran und Dioxan; aprotische dipolare Lösungsmittel wie Dimethylformamid, N-Methylpyrrolidon oder Tetramethylharnstoff. Auch Gemische dieser Stoffe können als Lösungs- und Verdünnungsmittel verwendet werden.

Die Reaktionszeit beträgt normalerweise 1 bis 20 Stunden, meist 2 bis 12 Stunden.

Das Produkt kann in üblicher Weise isoliert werden: Dazu kann das Rohprodukt in Wasser eingetragen und die Lösung bei Temperaturen von 10 bis 60 °C mit anorganischen Säuren auf pH 2 bis 6 eingestellt werden. Das ausgefallene Produkt kann anschließend durch Filtration isoliert werden. Als anorganische Säuren werden bevorzugt Salzsäure, Schwefelsäure oder Phosphorsäure verwendet.

Als Substituenten $R^1$ und $R^2$ und Indices n und m in Formel I kommen unabhängig voneinander für das Verfahren folgende Bedeutungen bevorzugt in Betracht:

$R^1$ und $R^2$

- unverzweigtes oder verzweigtes $C_1$-$C_{20}$-Alkyl, vorzugsweise unverzweigtes oder verzweigtes $C_1$-$C_{12}$-Alkyl, wie Methyl, Ethyl, n-Propyl, iso-Propyl, n-Butyl, iso-Butyl, sec,-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl,
- unverzweigtes oder verzweigtes $C_2$-$C_{20}$-Alkoxyalkyl, bevorzugt unverzweigtes oder verzweigtes $C_2$-$C_8$-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, n-Pentoxymethyl, iso-Pentoxymethyl, sec.-Pentoxymethyl, tert.-Pentoxymethyl, neo-Pentoxymethyl, 1,2-Dimethylpropoxymethyl, n-Hexoxymethyl, iso-Hexoxymethyl, sec.-Hexoxymethyl, n-Heptoxymethyl, iso-Heptoxymethyl, Methoxy-1-ethyl, Ethoxy-1-ethyl, n-Propoxy-1-ethyl, iso-Propoxy-1-ethyl, n-Butoxy-1-ethyl, iso-Butoxy-1-ethyl, sec.-Butoxy-1-ethyl, tert.-Butoxy-1-ethyl, n-Pentoxy-1-ethyl, iso-Pentoxy-1-ethyl, sec.-Pentoxy-1-ethyl, tert.-Pentoxy-1-ethyl, neo-Pentoxy-1-ethyl, 1,2-Dimethylpropoxy-1-ethyl, n-Hexoxy-1-ethyl, iso-Hexoxy-1-ethyl, sec.-Hexoxy-1-ethyl, Methoxy-2-ethyl, Ethoxy-2-ethyl, n-Propoxy-2-ethyl, iso-Propoxy-2-ethyl, n-Butoxy-2-ethyl, iso-Butoxy-2-ethyl, sec.-Butoxy-2-ethyl, tert.-Butoxy-2-ethyl, n-Pentoxy-2-ethyl, iso-Pentoxy-2-ethyl, sec.-Pentoxy-2-ethyl, tert.-Pentoxy-2-ethyl, neo-Pentoxy-2-ethyl, 1.2-Dimethyl-propoxy-2-ethyl, n-Hexoxy-2-ethyl, iso-Hexoxy-2-ethyl und sec.-Hexoxy-2-ethyl,
- $C_3$-$C_{12}$-Cycloalkyl, bevorzugt $C_3$-$C_8$-Cycloalkyl, wie Cyclopropyl, Cyclobutyl, Cyclopentyl, Cyclohexyl, Cycloheptyl und Cyclooctyl,
- Halogen, wie Fluor, Chlor, Brom und Jod, bevorzugt Chlor und Brom,

- Aryl, bevorzugt Phenyl, 1-Naphthyl, 2-Naphthyl, 1-Anthryl, 2-Anthryl und 9-Anthryl, besonders bevorzugt Phenyl,
- $C_7$-$C_{20}$-Alkylaryl, bevorzugt $C_7$-$C_{10}$-Alkylphenyl, wie 2-Methylphenyl, 3-Methylphenyl, 4-Methylphenyl, 2-Ethylphenyl, 3-Ethylphenyl, 4-Ethylphenyl, 2-n-Propylphenyl, 3-n-Propylphenyl, 4-n-Propylphenyl, 2-iso-Propylphenyl, 3-iso-Propylphenyl, 4-iso-Propylphenyl, 2-n-Butylphenyl, 3-n-Butylphenyl, 4-n-Butylphenyl, 2-iso-Butylphenyl, 3-iso-Butylphenyl, 4-iso-Butylphenyl, 2-sec.-Butylphenyl, 3-sec.-Butylphenyl, 4-sec.-Butylphenyl, 2-tert.-Butylphenyl, 3-tert.-Butylphenyl und 4-tert.-Butylphenyl,
- $C_7$-$C_{20}$-Alkoxyaryl, bevorzugt $C_7$-$C_{10}$-Alkoxyphenyl, wie 2-Methoxyphenyl. 3-Methoxyphenyl, 4-Methoxyphenyl, 2-Ethoxyphenyl, 3-Ethoxyphenyl, 4-Ethoxyphenyl, 2-n-Propoxyphenyl, 3-n-Propoxyphenyl, 4-n-Propoxyphenyl, 2-iso-Propoxyphenyl, 3-iso-Propoxyphenyl, 4-iso-Propoxyphenyl, 2-n-Butoxyphenyl, 3-n-Butoxyphenyl, 4-n-Butoxyphenyl, 2-iso-Butoxyphenyl, 3-iso-Butoxyphenyl, 4-iso-Butoxyphenyl, 2-sec.-Butoxyphenyl, 3-sec.-Butoxyphenyl, 4-sec.-Butoxyphenyl, 2-tert.-Butoxyphenyl, 3-tert.-Butoxyphenyl und 4-tert.-Butoxyphenyl,
- zwei- oder dreifach durch $C_1$-$C_8$-Alkyl substituiertes Aryl, bevorzugt zwei- oder dreifach durch $C_1$-$C_4$-Alkyl substituiertes Phenyl, wie 2,4-Dimethylphenyl, 3,4-Dimethylphenyl und 3,4,5-Trimethylphenyl,
- zwei- oder dreifach durch $C_1$-$C_8$-Alkoxy substituiertes Aryl, bevorzugt zwei- oder dreifach durch $C_1$-$C_4$-Alkoxy substituiertes Phenyl, wie 3,4-Dimethoxyphenyl und 3,4,5-Trimethoxyphenyl,
- ein- bis dreifach durch $C_1$-$C_4$-Halogenalkyl substituiertes Aryl, bevorzugt ein- bis dreifach durch $C_1$-$C_2$-Fluor- und Chloralkyl Phenyl, besonders bevorzugt ein- bis dreifach durch Trifluormethyl und Trichlorethyl, substituiertes Phenyl, wie 4-Trifluormethylphenyl und 4-Trichlormethylphenyl,
- ein- bis dreifach durch $C_1$-$C_4$-Halogenalkoxy substituiertes Aryl, bevorzugt ein- bis dreifach durch $C_1$-$C_2$-Fluor- und Chloralkoxy Phenyl, besonders bevorzugt ein- bis dreifach durch Trifluormethoxy und Trichlormethoxy substituiertes Phenyl, wie Trifluormethoxyphenyl,
- ein- bis dreifach durch Halogen substituiertes Aryl, bevorzugt ein- bis dreifach durch Fluor oder Chlor substituiertes Phenyl, wie 4-Chlorphenyl, 3,4-Dichlorphenyl, 4-Bromphenyl, 4-Fluorphenyl und 4-Fluor-3-chlorphenyl,
- $C_7$-$C_{20}$-Aryloxy, bevorzugt $C_7$-$C_{10}$-Phenylalkyl, wie Benzyl, Phenethyl, 1-Phenyl-n-propyl, 2-Phenyl-n-propyl, 3-Phenyl-n-propyl, 1-Phenyl-iso-propyl, 2-Phenyl-iso-propyl, 1-Phenyl-n-butyl, 2-Phenyl-n-butyl, 3-Phenyl-n-butyl, 4-Phenyl-n-butyl, 1-Phenyl-iso-butyl, 2-Phenyl-iso-butyl, 3-Phenyl-iso-butyl, 1-Phenyl-sec.-butyl, 1-Benzyl-n-propyl, 2-Phenyl-sec.-butyl, 3-Phenyl-sec.-butyl und 1,1-Dimethylphenethyl,
- im Arylteil ein- bis dreifach durch Halogen substituiertes $C_7$-$C_{20}$-Aryl-alkyl, bevorzugt im Phenylteil ein- bis dreifach durch Fluor oder Chlor $C_7$-$C_{10}$-Phenyl-alkyl, wie 4-Fluorbenzyl, 4-Chlorbenzyl, 2,4-Dichlorbenzyl und 3,4-Dichlorbenzyl,
- im Arylteil ein- bis dreifach durch $C_1$-$C_8$-Alkyl substituiertes $C_7$-$C_{20}$-Aryl-alkyl, bevorzugt im Phenylteil ein- bis dreifach durch $C_1$-$C_4$-Alkyl substituiertes $C_7$-$C_{10}$-Phenylalkyl, besonders bevorzugt im Phenylteil ein- bis dreifach durch $C_1$-$C_2$-Alkyl substituiertes $C_7$-$C_{10}$-Phenylalkyl, wie 4-Methylphenyl, 4-Ethylbenzyl und 4-Methylphenethyl,
- im Arylteil ein- bis dreifach durch $C_1$-$C_8$-Alkoxy substituiertes $C_7$-$C_{20}$-Aryl-alkyl, bevorzugt im Phenylteil ein- bis dreifach durch $C_1$-$C_4$-Alkoxy $C_7$-$C_{10}$-Phenylalkyl, besonders bevorzugt im Phenylteil ein- bis dreifach durch $C_1$-$C_2$-Alkoxy substituiertes $C_7$-$C_{10}$-Phenylalkyl, wie 4-Methoxybenzyl, 4-Ethoxybenzyl und 4-Methoxyphenethyl,
- im Arylteil ein- bis dreifach durch $C_1$-$C_4$-Halogenalkyl,substituiertes $C_7$-$C_{20}$-Aryl-alkyl, bevorzugt im Phenylteil ein- bis dreifach durch $C_1$-$C_2$-Fluor- und Chloralkyl substituiertes $C_7$-$C_{10}$-Phenylalkyl, besonders bevorzugt im Phenylteil ein- bis dreifach durch Trifluormethyl und Trichlormethyl substituiertes $C_7$-$C_{10}$-Phenylalkyl, wie 4-Trifluormethylbenzyl und 4-Trichlormethylbenzyl,
- im Arylteil ein- bis dreifach durch $C_1$-$C_4$-Halogenalkoxy substituiertes $C_7$-$C_{20}$-Aryl-alkyl, bevorzugt im Phenylteil ein- bis dreifach durch $C_1$-$C_2$-Halogenalkyl substituiertes $C_7$-$C_{10}$-Phenyl-alkyl, besonders bevorzugt ein- bis dreifach durch Trifluormethyl und Trichlormethyl substituiertes $C_7$-$C_{10}$-Phenylalkyl, wie 4-Trifluormethoxybenzyl und 4-Trichlormethoxybenzyl
- durch ein, zwei oder drei Phenoxygruppen substituiertes Phenyl,
- durch Halogen und $C_1$-$C_4$-Alkyl zwei- oder dreifach substituiertes Phenyl, wie 2-Methyl-4-chlorphenyl und 3-Methyl-4-fluorphenyl,
- durch Halogen und $C_1$-$C_4$-Alkoxy zwei- oder dreifach substituiertes Phenyl, wie 3-Chlor-4-methoxyphenyl,
- durch Halogen und $C_1$-$C_4$-Halogenalkyl zwei- oder dreifach substituiertes Phenyl, wie 2-Chlor-4-trifluormethylphenyl,
- durch Halogen und Phenoxy zwei- oder dreifach substituiertes Phenyl, wie 3-Chlor-4-phenoxyphenyl,

- durch $C_1$-$C_4$-Alkyl und $C_1$-$C_4$-Alkoxy zwei- oder dreifach substituiertes Phenyl, wie 2-Methyl-4-methoxyphenyl,
- durch $C_1$-$C_4$-Alkyl und $C_1$-$C_4$-Halogenalkyl zwei- oder dreifach substituiertes Phenyl, wie 3-Methyl-4-trichlormethylphenyl,
- durch $C_1$-$C_4$-Alkyl und Phenoxy zwei- oder dreifach substituiertes Phenyl, wie 2-Methyl-4-phenoxyphenyl,
- durch $C_1$-$C_4$-Alkoxy und $C_1$-$C_4$-Halogenalkyl zwei- oder dreifach substituiertes Phenyl, wie 3-Trifluormethyl-4-methoxyphenyl,
- durch $C_1$-$C_4$-Alkoxy und Phenoxy zwei- oder dreifach substituiertes Phenyl, wie 3-Methoxy-4-phenoxyphenyl,
- durch $C_1$-$C_4$-Halogenalkyl und Phenoxy zwei- oder dreifach substituiertes Phenyl, wie 3-Trifluormethyl-4-phenoxyphenyl,
- durch Halogen, $C_1$-$C_4$-Alkyl und $C_1$-$C_4$-Alkoxy dreifach substituiertes Phenyl,
- durch Halogen, $C_1$-$C_4$-Alkyl und $C_1$-$C_4$-Halogenalkyl dreifach substituiertes Phenyl,
- durch Halogen, $C_1$-$C_4$-Alkyl und Phenoxy dreifach substituiertes Phenyl,
- durch Halogen, $C_1$-$C_4$-Alkoxy und $C_1$-$C_4$-Halogenalkyl dreifach substituiertes Phenyl,
- durch Halogen, $C_1$-$C_4$-Alkoxy und Phenoxy dreifach substituiertes Phenyl,
- durch Halogen, $C_1$-$C_4$-Halogenalkyl und Phenoxy dreifach substituiertes Phenyl,
- durch $C_1$-$C_4$-Alkyl, $C_1$-$C_4$-Alkoxy und $C_1$-$C_4$-Halogenalkyl dreifach substituiertes Phenyl,
- durch $C_1$-$C_4$-Alkyl, $C_1$-$C_4$-Alkoxy und Phenoxy dreifach substituiertes Phenyl,
- durch $C_1$-$C_4$-Alkyl, $C_1$-$C_4$-Halogenalkyl und Phenoxy dreifach substituiertes Phenyl,
- durch $C_1$-$C_4$-Alkoxy, $C_1$-$C_4$-Halogenalkyl und Phenoxy dreifach substituiertes Phenyl,

$R^1$ und $R^2$ gemeinsam

- $(CH_2)_n$, wie $CH_2$, $(CH_2)_2$, $(CH_2)_3$, $(CH_2)_4$, $(CH_2)_5$ und $(CH_2)_6$, bevorzugt $(CH_2)_3$, $(CH_2)_4$, $(CH_2)_5$, $(CH_2)_6$, besonders bevorzugt $(CH_2)_3$ und $(CH_2)_4$,
- $(CH=CH)_m$, wie $(CH=CH)$, $(CH=CH)_2$, $(CH=CH)_3$, bevorzugt $(CH=CH)_2$), $(CH=CH)_3$ besonders bevorzugt $(CH=CH)_2$,

n

- 1 bis 6, vorzugsweise 3 bis 6, besonders bevorzugt 3 und 4,

m

- 1 bis 3, vorzugsweise 2 und 3, besonders bevorzugt 2.

Ein- bis dreifach substituiert bedeutet ein-, zwei- oder dreifach substituiert.

Für die Stoffe selbst sind von den aufgeführten Bedeutungen für das Verfahren diejenigen Verbindungen ausgeschlossen, in denen $R^1$ und $R^2$ für Halogen stehen, sowie in denen $R^1$ und $R^2$ gemeinsam $(CH=CH)_m$ ist.

Die erhaltenen Imidazol-2-carbonsäuren sind Zwischenprodukte z.B. zur Herstellung von Pharmazeutika oder von Farbstoffen (DE-A-34 28 493).

Herstellungsbeispiele

Beispiel 1

4,5-Dichlorimidazol-2-carbonsäure (Verbindung Nr. 1)

Eine Mischung aus 137 g (1 mol) 4,5-Dichlorimidazol, 414 g (3 mol) $K_2CO_3$ und 1000 ml flüssigem Kohlendioxid wurde in einem Autoklaven 10 Stunden bei 200 °C unter Eigendruck gerührt. Der feste Austrag wurde pulverisiert, in 2 l Wasser suspendiert und unter Eiskühlung mit konz. Salzsäure auf pH 3 bis 4 gestellt. Der ausgefallene Niederschlag wurde abfiltriert und aus Wasser umkristallisiert. Man erhielt 136 g (75 %) 4,5-Dichlorimidazol-2-carbonsäure (Verbindung Nr. 1), Smp. 240 °C (Zers.).

Analog Beispiel 1 wurden die folgenden Verbindungen hergestellt:

Tabelle 1

| Verbindung Nr. | Ausgangsprodukt | Mol Ausgangsprod. | Mol $K_2CO_3$ | Reaktionstemp. | Produkt | Smp. | Ausbeute |
|---|---|---|---|---|---|---|---|
| 2 | 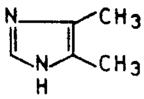 | 1 | 3 | 180°C | 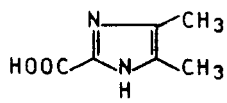 | 190°C Zers. | 68 % |
| 3 | 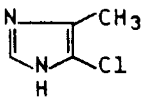 | 1 | 2 | 200°C | 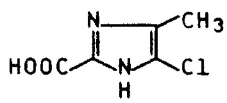 | 270°C Zers. | 66 % |

Beispiel 2
Benzimidazol-2-carbonsäure (Verbindung Nr. 4)

Eine Mischung aus 118 g (1 mol) Benzimidazol, 276 g (2 mol) $K_2CO_3$ und 400 ml flüssigem Kohlendioxid wurde in einem Autoklaven 10 Stunden bei 250 °C unter Eigendruck gerührt. Der feste Austrag wurde pulverisiert, in 2,5 l Wasser suspendiert und unter Eiskühlung mit konz. Salzsäure auf pH 3 bis 4 gestellt. Der ausgefallene Niederschlag wurde abfiltriert und aus Wasser umkristallisiert. Man erhielt 118 g (73 %) Benzimidazol-2-carbonsäure (Verbindung Nr. 4); Smp. 170 °C (Zers.).

Analog Beispiel 2 wurden die folgenden Verbindungen hergestellt.

Tabelle 2

| Verbindung Nr. | Ausgangsprodukt | Mol Ausgangsprod. | Mol $K_2CO_3$ | Reaktionstemp. | Produkt | Smp. | Ausbeute |
|---|---|---|---|---|---|---|---|
| 5 | 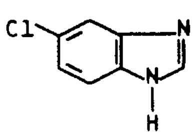 | 1 | 2 | 250°C | 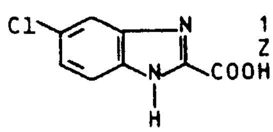 | 155°C Zers. | 55 % |
| 6 | 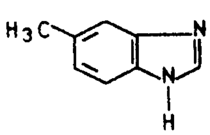 | 1 | 2 | 250°C | 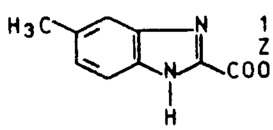 | 140°C Zers. | 50 % |

## Patentansprüche

1. Verfahren zur Herstellung von 4,5-disubstituierten Imidazol-2-carbonsäuren der allgemeinen Formel I

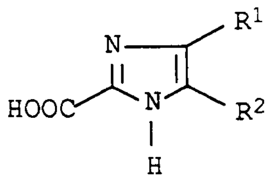 (I),

in der die Substituenten

$R^1$ und $R^2$ unabhängig voneinander $C_1$-$C_{20}$-Alkyl, $C_2$-$C_{20}$-Alkoxyalkyl, $C_3$-$C_{12}$-Cycloalkyl, Halogen, gegebenenfalls durch $C_1$-$C_4$-Alkyl, $C_1$-$C_4$-Alkyl, $C_1$-$C_4$-Alkoxy, Halogen, C1-C4-Halogenalkyl, $C_1$-$C_4$-Halogenalkoxy oder Phenoxy substituiertes Phenyl oder $C_7$-$C_{20}$-Phenylalkyl oder gemeinsam eine gegebenenfalls durch $C_1$-$C_4$-Alkyl, $C_1$-$C_4$-Alkoxy und/oder Halogen ein- bis zweifach substituierte $(CH_2)_n$- oder $(CH=CH)_m$-Gruppe, in der n für 1 bis 6 und m für 1 bis 3 steht,

bedeuten, dadurch gekennzeichnet, daß man 4,5-disubstituierte Imidazole der allgemeinen Formel II

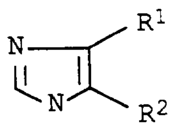 (II),

in der $R^1$ und $R^2$ die obengenannten Bedeutungen haben, mit Kohlendioxid bei Temperaturen von 150 bis 300 °C und Drücken von 10 bis 300 bar in Gegenwart von anorganischen Carbonaten, Hydrogencarbonaten und/oder Hydroxiden als Base umsetzt.

2. Verfahren zur Herstellung von 4,5-disubstituieren Imidizol-2-carbonsäuren nach Anspruch 1, dadurch gekennzeichnet, daß min die Umsetzung in Gegenwart von Alkalimetallhydrogencarbonaten, Alkali- oder Erdalkalimetallcarbonaten und/oder Alkali- oder Erdalkalimetallhydroxiden vornimmt.

3. Verfahren zur Herstellung von Imidazol-2-carbonsäuren I nach Anspruch 1, dadurch gekennzeichnet, daß man die Umsetzung bei Drücken von 10 bis 300 bar in Gegenwart von Alkalimetallcarbonaten, -hydrogen-carbonaten und/oder -hydroxiden vornimmt.

4. Verfahren zur Herstellung von Imidazol-2-carbonsäuren I nach Anspruch 1, dadurch gekennzeichnet. daß man die Umsetzung bei Drürken von 40 bis 180 bar und Temperaturen von 180 bis 280 °C in Gegenwart von 1 bis 5 Mol.% eines Alkalimetallcarbonates, -hydrogencarbonates und/oder -hydroxides vornimmt.

5. Verfahren zur Herstellung von Imidazol-2-carbonsäuron I nach Anspruch 1, dadurch gekennzeichnet, daß man die Umsatzung bei Drücken von 40 bis 180 bar und Temperaturen von 180 bis 280 °C in Gegenwart von 1 bis 3,5 Mol.% Kaliumcarbonat vornimmt.

## Claims

1. A process for preparing 4,5-disubstituted imidazole-2-carboxylic acids of the general formula I

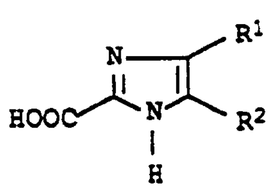 (I),

where the substitutents

$R^1$ and $R^2$ are each independently of the other $C_1$-$C_{20}$-alkyl, $C_2$-$C_{20}$-alkoxyalkyl, $C_3$-$C_{12}$-cycloalkyl, halogen or unsubstituted $C_1$-$C_4$-alkyl-, $C_1$-$C_4$-alkoxy-, halogen-, $C_1$-$C_4$-haloalkyl-, $C_1$-$C_4$-haloalkoxy- or phenoxy-substituted phenyl or $C_7$-$C_{20}$-phenylalkyl or are together unsubstituted or $C_1$-$C_4$-alkyl, $C_1$-$C_4$-alkoxy- and/or halogen-monosubstituted or -disubstituted $(CH_2)_n$ or $(CH=CH)_m$, where n is from 1 to 6 and m is from 1 to 3,

which comprises reacting a 4,5-disubstituted imidazole of the general formula II

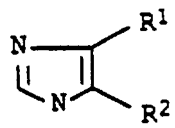

(II),

where $R^1$ and $R^2$ are as defined above, with carbon dioxide at temperatures from 150 to 300 °C pressures from 10 to 300 bar in the presence of inorganic carbonates, bicarbonates and/or hydroxides as base.

2. A process for preparing 4,5-disubstituted imidazole-2-carboxylic acids as claimed in claim 1, wherein the reaction is carried out in the presence of alkali metal bicarbonates, alkali metal or alkaline earth metal carbonates and/or alkali metal or alkaline earth metal hydroxides.

3. A process for preparing imidazole-2-carboxylic acids I as claimed in claim 1, wherein the reaction is carried out at pressures from 10 to 300 bar in the presence of alkali metal carbonates, bicarbonates and/or hydroxides.

4. A process for preparing imidazole-2-carboxylic acids I as claimed in claim 1, wherein the reaction is carried out at pressures from 40 to 180 bar and temperatures from 180 to 280 °C in the presence of from 1 to 5 mol % of an alkali metal carbonate, bicarbonate and/or hydroxide.

5. A process for preparing imidazole-2-carboxylic acids I as claimed in claim 1, wherein the reaction is carried out at pressures from 40 to 180 bar and temperatures from 180 to 280 °C in the presence of from 1 to 3.5 mol % of potassium carbonate.

## Revendications

1. Procédé de préparation d'acides imidazole-2-carboxyliques 4,5-disubstitués, répondant à la formule générale I

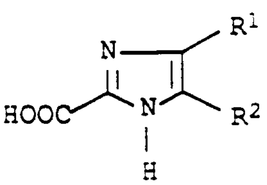

(I),

dans laquelle les substituants

$R^1$ et $R^2$ représentent, chacun indépendamment l'un de l'autre, un radical alkyle en $C_1$ à $C_{20}$, un radical alcoxy($C_2$-$C_{20}$)alkyle, un radical cycloalkyle en $C_3$ à $C_{12}$, un atome d'halogène, un radical phénylalkyle en $C_7$ à $C_{20}$, ou phényle, éventuellement substitué par des radicaux alkyle en $C_1$ à $C_4$, alcoxy en $C_1$ à $C_4$, des atomes d'halogènes, des atomes d'halogènes, des radicaux halogénoalkyle en $C_1$ à $C_4$, halogénoalcoxy en $C_1$ à $C_4$ ou phénoxy, ou forment, en commun, un radical $(CH=CH)_m$ ou $(CH_2)_n$, éventuellement substitué une ou deux fois par des radicaux alkyle en $C_1$ à $C_4$, alcoxy en $C_1$ à $C_4$ et/ou des atomes d'halogènes, n ayant une valeur de 1 à 6 et m une valeur de 1 à 3,

caractérisé en ce que l'on fait réagir des imidazoles 4,5-disubstitués de la formule générale II

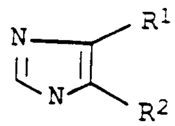

(II),

dans laquelle $R^1$ et $R^2$ possèdent les significations qui leur ont été attribuées ci-dessus, avec du dioxyde de carbone, à des températures de 150 à 300 °C et sous des pressions de 10 à 300 bars, en présence d'hydroxydes, d'hydrogénocarbonates et/ou de carbonates inorganiques, à titre de bases.

**2.** Procédé de préparation d'acides imidazole-2-carboxyliques 4,5-disubstitués suivant la revendication 1, caractérisé en ce que l'on entreprend la réaction en présence d'hydrogénocarbonates de métaux alcalins, de carbonates de métaux alcalins ou de métaux alcalinoterreux et/ou d'hydroxydes de métaux alcalins ou de métaux alcalino-terreux.

**3.** Procédé de préparation d'acides imidazole-2-carboxyliques I suivant la revendication 1, caractérisé en ce que l'on entreprend la réaction sous des pressions de 10 à 300 bars en présence de carbonates de métaux alcalins, d'hydrogénocarbonates de métaux alcalins et/ou d'hydroxydes de métaux alcalins.

**4.** Procédé de préparation d'acides imidazole-2-carboxyliques I suivant la revendication 1, caractérisé en ce que l'on entreprend la réaction sous des pressions de 40 à 180 bars et à des températures de 180 à 280 °C en présence de 1 à 5% molaires d'un carbonate de métal alcalin, d'un hydrogénocarbonate de métal alcalin et/ou d'un hydroxyde de métal alcalin.

**5.** Procédé de préparation d'acides imidazole-2-carboxyliques I suivant la revendication 1, caractérisé en ce que l'on entreprend la réaction sous des pressions de 40 à 180 bars et à des températures de 180 à 280 °C en présence de 1 à 3,5% molaires de carbonate de potassium.